# EUROPEAN PATENT SPECIFICATION

(11) **EP 2 572 725 B1**
(45) Date of publication and mention of the grant of the patent: **08.07.2015**
(21) Application number: 11007684.1
(22) Date of filing: 21.09.2011
(51) Int. Cl.: A61K 39/00

(54) **MSI-specific frameshift peptides (FSP) for prevention and treatment of cancer**
MSI-spezifische Frameshift-Peptide zur Vorbeugung und Behandlung von Krebs
Peptides à déphasage MSI spécifique pour la prévention ou le traitement du cancer

(43) Date of publication of application: 27.03.2013
(73) Proprietor: Ruprecht-Karls-Universität Heidelberg, 69117 Heidelberg (DE)
(72) Inventor: Kloor, Matthias, 67071 Ludwigshafen (DE); Reuschenbach, Miriam, 69124 Heidelberg (DE); Knebel-Doeberitz, Magnus, 69118 Heidelberg (DE)
(74) Representative: Schüssler, Andrea

(56) References cited:
- WO-A2-03/087162
- LINNEBACHER M ET AL: "Frameshift peptide-derived T-cell epitopes: a source of novel tumor-specific antigens", INTERNATIONAL JOURNAL OF CANCER, JOHN WILEY & SONS, INC, UNITED STATES, SWITZERLAND, GERMANY, vol. 93, no. 1, 1 July 2001 (2001-07-01), pages 6-11, XP002216743, ISSN: 0020-7136, DOI: 10.1002/IJC.1298
- SCHWITALLE YVETTE ET AL: "Immunogenic peptides generated by frameshift mutations in DNA mismatch repair-deficient cancer cells.", CANCER IMMUNITY 25 NOV 2004 LNKD- PUBMED:15563124, vol. 4, 25 November 2004 (2004-11-25), page 14, XP009159420, ISSN: 1424-9634
- SCHWITALLE Y ET AL: "Immune Response Against Frameshift-Induced Neopeptides in HNPCC Patients and Healthy HNPCC Mutation Carriers", GASTROENTEROLOGY, ELSEVIER, PHILADELPHIA, PA, vol. 134, no. 4, 1 April 2008 (2008-04-01) , pages 988-997, XP022584833, ISSN: 0016-5085, DOI: 10.1053/J.GASTRO.2008.01.015 [retrieved on 2008-01-11]
- RIPBERGER EVA ET AL: "Identification of an HLA-A0201-restricted CTL epitope generated by a tumor-specific frameshift mutation in a coding microsatellite of the OGT gene.", JOURNAL OF CLINICAL IMMUNOLOGY SEP 2003 LNKD- PUBMED:14601650, vol. 23, no. 5, September 2003 (2003-09), pages 415-423, XP002676134, ISSN: 0271-9142
- MIRIAM REUSCHENBACH ET AL: "Serum antibodies against frameshift peptides in microsatellite unstable colorectal cancer patients with Lynch syndrome", FAMILIAL CANCER, KLUWER ACADEMIC PUBLISHERS, DO, vol. 9, no. 2, 2 December 2009 (2009-12-02), pages 173-179, XP019818173, ISSN: 1573-7292
- KLOOR MATTHIAS ET AL: "Immune evasion of microsatellite unstable colorectal cancers.", INTERNATIONAL JOURNAL OF CANCER. JOURNAL INTERNATIONAL DU CANCER 1 SEP 2010 LNKD- PUBMED:20198617, vol. 127, no. 5, 1 September 2010 (2010-09-01), pages 1001-1010, XP002676135, ISSN: 1097-0215
- TOUGERON DAVID ET AL: "Tumor-infiltrating lymphocytes in colorectal cancers with microsatellite instability are correlated with the number and spectrum of frameshift mutations.", MODERN PATHOLOGY : AN OFFICIAL JOURNAL OF THE UNITED STATES AND CANADIAN ACADEMY OF PATHOLOGY, INC SEP 2009 LNKD- PUBMED:19503063, vol. 22, no. 9, September 2009 (2009-09), pages 1186-1195, XP002676136, ISSN: 1530-0285
- KIMURA TAKASHI ET AL: "MUC1 vaccine for individuals with advanced adenoma of the colon: a cancer immunoprevention feasibility study.", CANCER PREVENTION RESEARCH (PHILADELPHIA, PA.) JAN 2013, vol. 6, no. 1, January 2013 (2013-01), pages 18-26, ISSN: 1940-6215
- DATABASE MEDLINE [Online] US NATIONAL LIBRARY OF MEDICINE (NLM), BETHESDA, MD, US 20 January 2005 POPAT S ET AL: 'Systematic review of microsatellite instability and colorectal cancer prognosis.' Database accession no. NLM15659508
- DATABASE MEDLINE [Online] US NATIONAL LIBRARY OF MEDICINE (NLM), BETHESDA, MD, US February 2004 CARETHERS JOHN M ET AL: 'Use of 5-fluorouracil and survival in patients with microsatellite-unstable colorectal cancer.' Database accession no. NLM14762775

## Description

The present invention provides a vaccine for prevention and treatment of cancer characterized by microsatellite instability (MSI). The vaccine contains a combination of MSI-specific frameshift peptides (FSP) generating humoral and cellular responses against tumor cells or a nucleic acid encoding said FSP.

Human tumors develop through two major pathways of genome instability, chromosomal instability and microsatellite instability (MSI) that results from defects in the DNA mismatch repair system. MSI is encountered in 15% of colorectal cancers and a variety of extracolonic malignancies showing a deficient DNA mismatch repair system, including endometrial cancers, gastric cancers, small bowel cancers and tumors of other organs. MSI cancers may develop sporadically or in the context of a hereditary tumor syndrome, hereditary non-polyposis colorectal cancer (HNPCC) or Lynch syndrome.

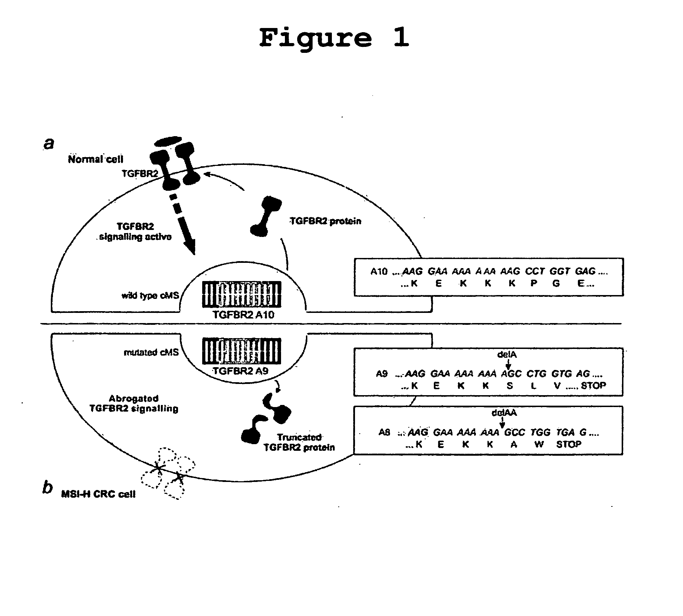

MSI colorectal cancers are characterized by a high immunogenicity that results from the generation of numerous frameshift peptides (FSP) during the development of MSI tumors as a direct result of mismatch repair deficiency leading to alterations of the translational reading frame when microsatellites in gene-encoding regions are affected by mutation (Figure 1).

The abundance of predictable MSI-specific FSP antigens and the fact that they directly result from the malignant transformation process render FSP highly promising targets for immune therapy. It is believed that the human immune system is a potential resource to eradicate tumor cells and that effective treatment can be developed if the components of the immune system are properly stimulated to recognize and eliminate cancer cells. Thus, immunotherapy, which comprises compositions and methods to activate the body's immune system, either directly or indirectly, to shrink or eradicate cancer, has been studied for many years as an adjunct to conventional cancer therapy.

WO03/087162-A2 discloses preventive and therapeutic vaccines involving FSP, directed i.a. to the treatment of HPNCC.

It is generally admitted that the growth and metastasis of tumors depends largely on their capacity to evade host immune surveillance. Most tumors express antigens that can be recognized to a variable extent by the host immune system, but in many cases, the immune response is inadequate. Failure to elicit a strong activation of effector T-cells may result from the weak immunogenicity of tumor antigens or inappropriate or absent expression of co-stimulatory molecules by tumor cells. For most T-cells, production of IL-2 and proliferation require a co-stimulatory signal simultaneous with TCR engagement, otherwise, T-cells may enter a functionally unresponsive state, known as clonal anergy.

In spite of the length of time that these therapies have been investigated, there remains a need for improved strategies for enhancing the immune response against the tumor antigens.

Nevertheless, there is a need in the art for safe and effective compositions that can stimulate the immune system as a cancer immunotherapy.

According to the invention, safe and effective stimulation of the immune system as a cancer immunotherapy is achieved by the subject matters defined in the claims. In vitro data showed that FSP are highly immunogenic and can elicit pronounced FSP-specific T cell responses in vitro (Linnebacher et al. 2001, Ripberger et al. 2003, Schwitalle et al. 2004). In further studies examining peripheral blood drawn from patients with MSI colon cancer, a high frequency of FSP-specific T cell responses was demonstrated (Schwitalle et al. 2008). In spite of the high number of FSP-specific T cells in the tumor and in the peripheral blood, these patients showed no signs of autoimmunity, suggesting that FSP vaccination approaches are not expected to have side effects in terms of autoimmunity.

Immunological analyses in individuals carrying a germ line mutation in DNA mismatch repair genes predisposing to hereditary non-polyposis colorectal cancer (HNPCC) were also found to exhibit cellular immune responses against FSP, even in the absence of a clinically detectable tumor. This suggests that FSP-specific immune responses may be protective in HNPCC individuals, suggesting that FSP vaccination may also be used in a preventive setting as the first specific prevention approach in an inherited cancer condition.

### In summary:

(a) Frameshift peptides (FSP) are MSI-specific and directly result from MSI tumor pathogenesis;
(b) No clinically relevant side effects are expected;
(c) Combinations of FSPs are predicted to target all tumors with MSI;
(d) FSP vaccination has been designed for therapy of 15% of colon cancers and tumors of the endometrium, stomach, small intestine and other organs;
(e) Molecular tumor analysis can identify patients that may benefit from FSP vaccination (targeted therapy); and
(f) FSP vaccination may be used as a preventive vaccination in high risk groups.

### Brief description of the drawings

Figure 1: Schematic illustration of coding microsatellite instability resulting from DNA mismatch repair deficiency (Kloor et al., 2010)
   Truncated proteins encompassing FSP sequences (red) are generated when coding microsatellite mutations lead to alterations of the translational reading frame (example: TGFBR2 protein).
Figure 2: Exemplary T cell responses against newly designed FSPs in peripheral blood from three patients with MSI colon cancer
Figure 3: Humoral immune responses against FSPs derived from AIM2(-1), HT001(-1), TAF1B(-1), and TGFBR2(-1)
   ELISA revealed FSP-specific antibody responses directed against neopeptides derived from AIM2(-1), HT001(-1), TAF1B(-1), and TGFBR2(-1). Peptide specificity was demonstrated by preabsorption of respective serum antibodies as described previously (Reuschenbach et al., 2008).
Figure 4: Cytotoxic responses as determined by CD107a surface expression
   **(A)** Significant FSP-specific responses were observed in different healthy donors after stimulation of T cells with the antigen for four weeks. Responses did only occur in the presence of antigen-presenting B cells and the FSP antigen. Representative responses are shown in bar graphs.
   **(B)** representative FACS analysis of CD107a assay for T cells incubated with B cells as antigen-presenting cells in the absence (left panel) or presence (right panel) of the FSP antigen HT001(-1).

Thus, the present invention provides a vaccine containing a combination of MSI tumor specific frameshift peptides (FSP), derived from TAF1B (Acc.No. L39061), HT001 (Acc.No. AF113539) and AIM2 (Acc.No. AF024714), and, optionally, in addition from TGFBR2 (Acc.No. NM_003242) or a nucleic acid encoding said FSP wherein said FSP arecapable of eliciting an immune response against cancer showing MSI.

The vaccine of the present invention contains a combination of FSP comprising or consisting of the following amino acid sequences:
NTQIKALNRGLKKKTILKKAGIGMCVKVSSIFFINKQKP (TAF1B(-1));
EIFLPKGRSNSKKKGRRNRIPAVLRTEGEPLHTPSVGMRETTGLGC (HT001(-1));
HSTIKVIKAKKKHREVKRTNSSQLV (AIM2(-1));
   and, optionally, in addition
ASPKCIMKEKKSLVRLSSCVPVALMSAMTTSSSQKNITPAILTCC (TGFBR2(-1)).

An immune response is defined as a condition fulfilling at least one of the following criteria: 1. The induction of CD8-positive T cells, as detectable by cytotoxicity assays or IFN-gamma secretion or perforin expression or granzyme B expression or other cytokines that may be produced by CD8-positive T cells, measurable as above background by ELISpot or intracellular cytokine staining or cytokine ELISA or equivalent methods. 2. The induction of CD4-positive T cells, as detectable by cytokine secretion measurable as above background by ELISpot or intracellular cytokine staining or cytokine ELISA or equivalent methods. Cytokines may comprise IFN-alpha, IFN-gamma, IL-2, IL-4, IL-5, IL-6, IL-10, IL-12, IL-13, IL-17, TNF-alpha, TGF-beta or other cytokines that may be produced by CD4-positive T cells. 3. The induction of antibodies, as detectable by Western blot, ELISA and equivalent or related methods. 4. The induction of any kind of cellular Immune response not mediated by CD8-positive or CD4-positive T cells as described in 1 and 2.

In a more preferred embodiment, the vaccine of the present invention additionally comprises an adjuvant and/or immunostimulatory cytokine or chemokine.

Suitable adjuvants include an aluminium salt such as aluminium hydroxide gel (alum) or aluminium phosphate, but may also be a salt of calcium, iron or zinc, or may be an insoluble suspension of acylated tyrosine, or acylated sugars, cationically or anionicaily derivatised polysaccharides, or polyphosphazenes. Other known adjuvants include CpG containing oligonucleotides. The oligonucleotides are characterised in that the CpG dinucleotide is unmethylated. Such oligonucleotides are well known and are described in, for example WO 96/02555.

The use of immunostimulatory cytokines has become an increasingly promising approach in cancer immunotherapy. The major goal is the activation of tumour-specific T lymphocytes capable of rejecting tumour cells from patients with low tumour burden or to protect patients from a recurrence of the disease. Strategies that provide high levels of immunostimulatory cytokines locally at the site of antigen have demonstrated pre-clinical and clinical efficacy. Preferred immunostimulatory cytokines comprise IL-2, IL-4, IL-7, IL-12, IFNs, GM-CSF and TNF-α.

Chemokines are small (7-16 kD), secreted, and structurally related soluble proteins that are involved in leukocyte and dendritic cell chemotaxis, PMN degranulation, and dendritic cell chemotaxis, PMN degranulation, and angiogenesis. Chemokines are produced during the initial phase of host response to injury, allergens, antigens, or invading microorganisms. Chemokines selectively attract leukocytes to inflammatory foci, inducing both cell migration and activation. Chemokines may enhance innate or specific host immunity against tumors and, thus may also be useful in combination with an FSP.

The vaccine of the present invention might also contain a nucleic acid encoding the FSP for DNA immunization, a technique used to efficiently stimulate humoral and cellular immune responses to protein antigens. The direct injection of genetic material into a living host causes a small amount of its cells to produce the introduced gene products. This inappropriate gene expression within the host has important immunological consequences, resulting in the specific immune activation of the host against the gene delivered antigen. Direct injection of naked plasmid DNA induces strong immune responses to the antigen encoded by the gene vaccine. Once the plasmid DNA construct is injected the host cells take up the foreign DNA, expressing the viral gene and producing the FSP inside the cell. This form of antigen presentation and processing induces both MHC and class I and class II restricted cellular and humoral immune responses. The DNA vaccines are composed of vectors normally containing two unites: the antigen expression unit composed of promoter/enhancer sequences, followed by antigen (FSP)-encoding and polyadenylation sequences and the production unit composed of sequences necessary for vector amplification and selection. The construction of vectors with vaccine inserts is accomplished using recombinant DNA technology and the person skilled in the art knows vectors that can be used for this approach. The efficiency of DNA immunization can be improved by stabilising DNA against degradation, and increasing the efficiency of delivery of DNA into antigen presenting cells. This has been demonstrated by coating biodegradable cationic microparticles (such as poly(lactide-co-glycolide) formulated with cetyltrimethylammonium bromide) with DNA. Such DNA-coated microparticles can be as effective at raising CTL as recombinant vaccinia viruses, especially when mixed with alum. Particles 300 nm in diameter appear to be most efficient for uptake by antigen presenting cells.

A variety of expression vectors, e.g., plasmids or viral vectors, may be utilised to contain and express nucleic acid sequences encoding an FSP of the present invention.

A preferred viral vector is a poxvirus, adenovirus, retrovirus, herpesvirus or adeno-associated virus (AAV). Particularly preferred poxviruses are a vaccinia virus, NYVAC, avipox virus, canarypox virus, ALVAC, ALVAC(2), fowlpox virus or TROVAC.

Recombinant alphavirus-based vectors have also been used to improve DNA vaccination efficiency. The gene encoding the FSP is inserted into the alphavirus replicon, replacing structural genes but leaving non-structural replicase genes intact. The Sindbis virus and Semliki Forest virus have been used to build recombinant alphavirus replicons. Unlike conventional DNA vaccinations, however, alphavirus vectors are only transiently expressed. Alphavirus replicons raise an immune response due to the high levels of protein expressed by this vector, replicon-induced cytokine responses, or replicon-induced apoptosis leading to enhanced antigen uptake by dendritic cells.

In a further preferred embodiment, the FSP contains a Tag sequence, preferably at the C-terminus which might be useful for purification of a recombinantly produced FSP. A preferred Tag sequence is a His-Tag. A particularly preferred His-Tag consists of 6 His-residues.

The vaccine of the present invention is administered in an amount suitable for immunization of an individual and, preferably, additionally contains one or more common auxiliary agents. The employed term "amount suitable for immunization of an individual" comprises any amount of FSP with which an individual can be immunized. The amount depends on whether immunization is intended as a prophylactic or therapeutic treatment. In addition, the individual's age, sex and weight play a role for determining the amount. Thus, the amount suitable for immunization of an individual refers to amounts of the active ingredients that are sufficient to affect the course and the severity of the tumor, leading to the reduction or remission of such pathology. An ""amount suitable for immunization of an individual" may be determined using methods known to one skilled in the art (see for example, Fingl et al., 1975). The term "individual" as used herein comprises an individual of any kind and being able to fall ill with carcinomas. Examples of such individuals are humans and animals as well as cells thereof.

The administration of the vaccine by injection may be made at various sites of the individual intramuscularly, subcutaneously, intradermally or in any other form of application. It may also be favourable to carry out one or more "booster injections" having about equal amounts.

The employed term "common auxiliary agents" comprises any auxiliary agents suitable for a vaccine to immunize an individual. Such auxiliary agents are, e.g., buffered common salt solutions, water, emulsions, such as oil/water emulsions, wetting agents, sterile solutions, etc.

An FSP, nucleic acid sequence or vector of the present invention can be present in the vaccine as such or in combination with carriers. It is favourable for the carriers in the individual not to be immunogenic. Such carriers may be the individual's own proteins or foreign proteins or fragments thereof. Carriers, such as serum albumin, fibrinogen or transferrin or a fragment thereof are preferred.

The vaccine of the present invention may be therapeutic, that is, the compounds are administered to treat an existing cancer, or to prevent the recurrence of a cancer, or prophylactic, that is, the compounds are administered to prevent or delay the development of cancer. If the compositions are used therapeutically, they are administered to cancer patients and are designed to elicit an immune response to stabilize a tumor by preventing or slowing the growth of the existing cancer, to prevent the spread of a tumor or of metastases, to reduce the tumor size, to prevent the recurrence of treated cancer, or to eliminate cancer cells not killed by earlier treatments. A vaccine used as a prophylactic treatment is administered to individuals who do not have cancer, and are designed to elicit an immune response to target potential cancer cells.

The present invention also relates to the use of FSP , nucleic acid sequence or vector as defined above for the production of a vaccine for the prevention of a carcinoma, e.g., preventive vaccination of a high risk group, or treatment of a carcinoma. For example, these may be a colorectal cancer, preferably a hereditary non-polyposis colorectal cancer (HNPCC), an endometrial cancer, a gastric cancer or small bowel cancer.

By means of the present invention it is possible to immunize individuals, in particular humans and animals. Immunization takes place by both induction of antibodies and stimulation of CD8⁺ T cells. Thus, it is possible to take prophylactic and therapeutic steps against carcinomas.

The below examples explain the invention in more detail.

### Example 1

### Detection of FSP-specific T cells in peripheral blood from patients with MSI colon cancer and healthy HNPCC mutation carriers

### (A) Methods (ELISpot Assay)

Frequencies of FSP-specific peripheral T cells (pTc) were quantified by determining the number of specific IFN-gamma-secreting Tc against newly designed FSPs derived from 3 cMS-containing candidate genes using ELISpot analysis. ELISpot assays were performed using 96-well nitrocellulose plates (Multiscreen; Millipore, Bedford, MA) coated overnight with mouse anti-human IFN-gamma monoclonal antibodies (mAb) (Mabtech, Nacka, Sweden) and blocked with serum containing medium. PTc (day 0, 1x10⁵/well) were plated 6-fold with autologous CD40-activated B cells (4x10⁴/well, TiBc or pBc, respectively) as antigen-presenting cells in 200 µl IMDM with 10% human AB serum. Peptides were added at a final concentration of 10 µg/mL. As a positive control, pTc were treated with 20 nmol/L phorbol-12-myristate-13-acetate in combination 350 nmol/L ionomycin. After incubation for 24 hours at 37°C, plates were washed thoroughly, incubated with biotinylated rabbit anti-human IFN-gamma mAb for 4 hours, washed again, and incubated with streptavidin-alkaline phosphatase for 2 hours, followed by a final wash step. Spots were detected by incubation with NBT/BCIP (Sigma-Aldrich) for 1 hour, reaction was stopped with water, and, after drying, spots were counted microscopically. Methods have been described in detail in Schwitalle et al., 2008.

### (B) Results

To examine whether FSP-specific T cell responses were detectable in the peripheral blood of MSI-H CRC patients, ELISpot analyses were performed. Autologous pBc, which showed high expression of MHC class I and II, and costimulators (CD40, CD80, and CD86) as well as B-cell-specific antigens (CD19 and CD23) were used as antigen-presenting cells.

Pronounced reactivities were observed against newly designed FSPs derived from AIM2(-1), HT001(-l), TAF1B(-1), and TGFBR2(-1):
TAF1B(-1) NTQIKALNRGLKKKTILKKAGIGMCVKVSSIFFINKQKP
HT001(-1) EIFLPKGRSNSKKKGRRNRIPAVLRTEGEPLHTPSVGMRETTGLGC
AIM2(-1) HSTIKVIKAKKKHREVKRTNSSQLV
TGFBR2(-1) ASPKCIMKEKKSLVRLSSCVPVALMSAMTTSSSQKNITPAILTCC
   (neopeptide sequences are underlined)

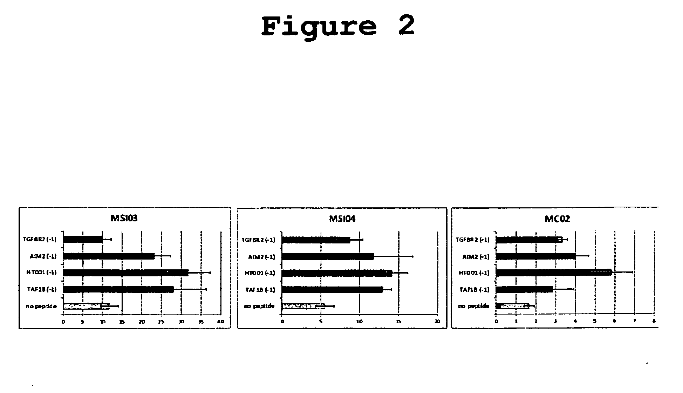

The results obtained from patients (n=8) are summarized in Table 1. Representative ELISpot results are shown in Figure 2.

**Table 1**

| **FSP-specific T cell responses against FSPs** | | | | | |
|---|---|---|---|---|---|
| Patient ID | no peptide | TAF1B (-1) | HT001 (-1) | AIM2 (-1) | TGFBR2 (-1) |
| **MSI01** | 4 | 8.17 | 5.83 | 3.5 | 6 |
| **MSI02** | 1.5 | 4.83 | 7.33 | 7.17 | 1.2 |
| **MSI03** | 11.83 | 28.17 | 31.83 | 23.33 | 10.16 |
| **MSI04** | 5.5 | 13 | 14.17 | 11.83 | 8.75 |
| **MSI05** | 3 | 7.75 | 16 | 10 | 2.8 |
| **MSI06** | 3.17 | 12 | 12.33 | 13.83 | 5.2 |
| **MSI07** | 6 | 17 | 16.83 | 14.5 | 9.33 |
| | | | | | |
| **MC01** | 1.17 | 3.5 | 3.5 | 3 | 8 |
| **MC02** | 1.67 | 2.83 | 5.83 | 4 | 3.33 |
| **MC03** | 0.83 | 1.17 | 1.17 | 2.17 | 4.4 |
| **MC04** | 15.17 | 18.83 | 18.67 | 15.17 | 20.75 |
| **MC05** | 0.33 | 3.17 | 2.17 | 1.33 | 5.67 |
| **MC06** | 30.5 | 37.83 | 37.8 | 34.83 | 39.5 |

Mean spot numbers from replicate analyses are given for each peptide and tested individual. MSI01-MSI07 - patients with MSI-H CRC, MC01-MC06 - healthy HNPCC germ line mutation carrier.

### Example 2

### Detection of FSP-specific humoral immune responses in peripheral blood from patients with MSI colon cancer and healthy HNPCC mutation carriers

### (A) Methods (ELISA)

For enzyme-linked immunosorbent assay (ELISA), peptides were coated to 96 well polystyrol microtiter plates "Maxisorp"' (Nunc, Roskilde, Denmark) at a concentration of 40 µg/ml in PBS overnight at 4 °C. After coating, plates were washed 4 times with PBS (0.05% Tween) and blocked for 1 h with 0.5% casein in PBS. Peptide binding to the microtiter plates and optimal saturating peptide concentration were assessed using an alkaline phosphatase-peptide competition assay. To monitor individual background reactivity of each serum, a control peptide derived from the p16^{INK4a} protein (p16_76-105) was used, against which no antibody reactivity was found in a large cohort of individuals (Reuschenbach et al., 2008). Each serum was diluted 1:100 in blocking buffer (0.5% casein in PBS) and tested in duplicates for the presence of antibodies against all FSPs and the control peptide. As a reference for inter-plate variance, one control serum was included on every plate, and peptide specific ODs of the control serum were used for normalization. Diluted sera (50 µl /well) were incubated for 1 h, and after a wash step plates were incubated with HRP-labeled rabbit anti-human-IgG antibody (Jackson Immunoresearch, West Grove, PA; 1:10,000 in blocking buffer) for 1 h. After washing, 50 µl /well of TMB substrate (Sigma, Deisenhofen, Germany) was added and the enzyme reaction was stopped after 30 minutes by adding 50 µl /well of 1 N H₂SO₄. Absorption was measured at 450 nm (reference wavelength 595 nm). Pre-absorption of serum antibodies for specificity control was done according to the method described in detail in Reuschenbach et al., 2008.

### (B) Results

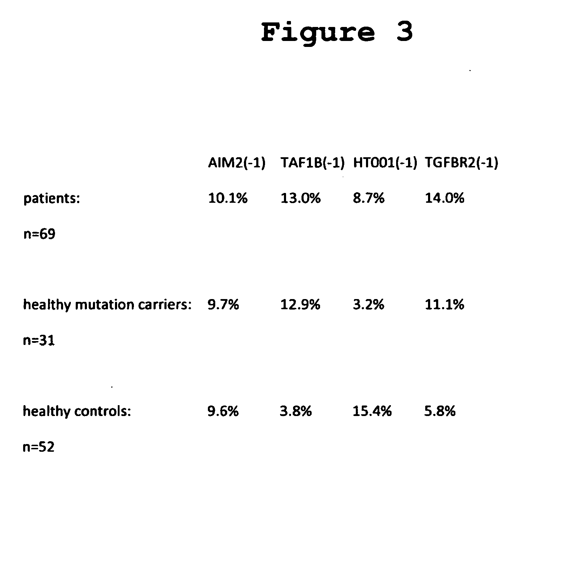

To examine whether FSP-specific antibodies were detectable in the peripheral blood of MSI-H CRC patients, healthy Lynch syndrome mutation carriers, and healthy controls ELISA analyses were performed. Pronounced reactivities were observed against newly designed FSPs derived from AIM2(-1), HT001(-1), TAF1B(-1), and TGFBR2(-1). ELISA results are shown in Figure 3.

### Example 3

### Detection of FSP-specific cytotoxic T cell responses

CD107a surface expression on T effector cells upon stimulation with the clinical FSP antigens was measured. CD107a assays are used to demonstrate secretion of cytotoxic granula containing perforin/granzyme B from effector cells. CD107a molecules are expressed on the surface of cytotoxic granula and become detectable on the cell surface if granula are released in the context of a cytotoxic T cell response.

To determine the potential of the FSP peptides to induce a cytotoxic cellular immune response, blood was drawn from healthy donors, and T cells were stimulated with the FSPs using dendritic cells as antigen-presenting cells. Stimulation was repeated weekly and over a time span of four weeks. After four weeks, T cells were harvested and coincubated with target cells and FSPs CD107a assay was used to analyze peptide-specific induction of a cytotoxic T cell response.

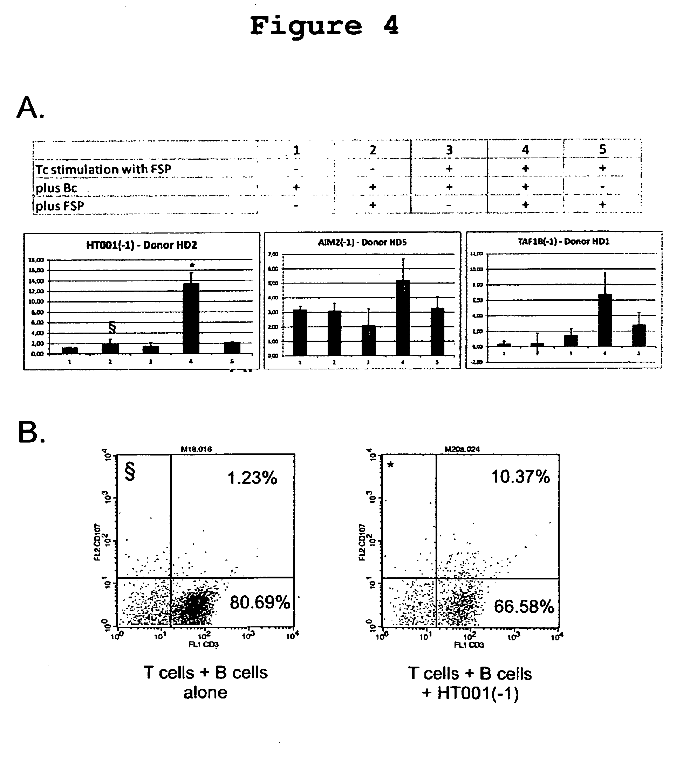

Cytotoxic responses as determined by CD107a surface expression were observed for T cells coincubated with B cells as antigen-presenting cells in the presence of the antigenic FSP (Figure 4A). Significant responses were observed in different healthy donors after stimulation of T cells with the antigen for four weeks. Representative responses are shown in bar graphs. Figure 4B shows representative FACS analysis of CD107a assay for T cells incubated with B cells as antigen-presenting cells in the absence (left panel) or presence (right panel) of the FSP antigen HT001(-1).

### References

Cunningham and Wells, Science 244 (1989), 1081-1085.
Fingl et al., The Pharmocological Basis of Therapeutics, Goodman and Gilman, eds. Macmillan Publishing Co., New York, pp. 1-46 (1975).
Kloor M, Michel S, von Knebel Doeberitz M. Immune evasion of microsatellite unstable colorectal cancers.
Int J Cancer. 2010 Mar 2. [Epub ahead of print]
Linnebacher M, Gebert J, Rudy W, Woerner S, Yuan YP, Bork P, von Knebel Doeberitz M. Frameshift peptide-derived T-cell epitopes: a source of novel tumor-specific antigens. Int J Cancer. 2001 Jul 1; 93(1):6-11.
Reuschenbach M, Waterboer T, Wallin KL, Einenkel J, Dillner J, Hamsikova E, Eschenbach D, Zimmer H, Heilig B, Kopitz J, Pawlita M, von Knebel Doeberitz M, Wentzensen N. Characterization of humoral immune responses against p16, p53, HPV16 E6 and HPV16 E7 in patients with HPV-associated cancers.
Int J Cancer. 2008 Dec 1;123(11):2626-31.
Reuschenbach M, Kloor M, Morak M, Wentzensen N, Germann A, Garbe Y, Tariverdian M, Findeisen P, Neumaier M, Holinski-Feder E, von Knebel Doeberitz M.
Serum antibodies against frameshift peptides in microsatellite unstable colorectal cancer patients with Lynch syndrome.
Fam Cancer. 2009 Dec 2. [Epub ahead of print]
Ripberger E, Linnebacher M, Schwitalle Y, Gebert J, von Knebel Doeberitz M. Identification of an HLA-A0201-restricted CTL epitope generated by a tumor-specific frameshift mutation in a coding microsatellite of the OGT gene. J Clin Immunol. 2003 Sep;23(5):415-23.
Schwitalle Y, Kloor M, Eiermann S, Linnebacher M, Kienle P, Knaebel HP, Tariverdian M, Benner A, von Knebel Doeberitz M. Immune response against frameshift-induced neopeptides in HNPCC patients and healthy HNPCC mutation carriers. Gastroenterology. 2008 Apr;134(4):988-97.
Schwitalle Y, Linnebacher M, Ripberger E, Gebert J, von Knebel Doeberitz M. Immunogenic peptides generated by frameshift mutations in DNA mismatch repair-deficient cancer cells. Cancer Immun. 2004 Nov 25;4:14.

## Claims

1. A vaccine containing a combination of MSI-specific frameshift peptides (FSP) comprising the following amino acid sequences:
NTQIKALNRGLKKKTILKKAGIGMCVKVSSIFFINKQKP (TAF1B (-1));
EIFLPKGRSNSKKKGRRNRIPAVLRTEGEPLHTPSVGMRETTGLGC (HT001 (-1)); and
HSTIKVIKAKKKHREVKRTNSSQLV (AIM2 (-1)).

2. The vaccine of claim 1 additionally containing an FSP comprising the following amino acid sequence:
ASPKCIMKEKKSLVRLSSCVPVALMSAMTTSSSQKNITPAILTCC (TGFBR2 (-1)).

3. The vaccine of claim 1 or 2, wherein the FSP additionally contains a Tag sequence.

4. A vaccine containing (i) the nucleic acid sequences encoding the FSP of any one of claims 1 to 3 or (ii) vector(s) containing said nucleic acid sequences.

5. The vaccine of claim 4, wherein said vector is a plasmid or viral vector.

6. The vaccine of claim 5, wherein said viral vector is a poxvirus, adenovirus, retrovirus, herpesvirus, alphavirus-based vector or adeno-associated virus (AAV).

7. The vaccine of claim 6, wherein said poxvirus is a vaccinia virus, NYVAC, avipox virus, canarypox virus, ALVAC, ALVAC(2), fowlpox virus or TROVAC.

8. The vaccine of any one of claims 1 to 7 additionally comprising an adjuvant and/or immunostimulatory cytokine or chemokine.

9. The vaccine of any one of claims 1 to 8, wherein the active compound is presented in an oil-in-water or a water-in-oil emulsion vehicle.

10. The vaccine of any one of claims 1 to 9 additionally comprising one or more other antigens.

11. The vaccine according to any one of claims 1 to 10 for use in a method for the prevention or treatment of a tumor.

12. Use of the MSI tumor specific frameshift peptides (FSP) as defined in any one of claims 1 to 3, the nucleic acid as defined in claim 4, or the vector as defined in any one of claims 4 to 7 for the preparation of a vaccine for the prevention or treatment of a tumor.

13. The vaccine of any of claims 1-11 for the use of claim 11 or 12 by preventive vaccination of high risk groups.

14. The vaccine of any of claims 1-11 for the use according to any one of claims 11 to 13, wherein the tumor is colorectal cancer, endometrial cancer, gastric cancer or small bowel cancer.

15. The vaccine of any of claims 1-11 for the use according to claim 14, wherein the colorectal cancer is hereditary non-polyposis colorectal cancer (HNPCC).

## Patentansprüche

1. Impfstoff, enthaltend eine Kombination aus MSI-spezifischen Frameshift-Peptiden (FSP), umfassend die folgenden Aminosäuresequenzen:
NTQIKALNRGLKKKTILKKAGIGMCVKVSSIFFINKQKP (TAF1B (-1));
EIFLPKGRSNSKKKGRRNRIPAVLRTEGEPLHTPSVGMRETTGLGC (HT001 (-1)); und
HSTIKVIKAKKKHREVKRTNSSQLV (AIM2 (-1)).

2. Impfstoff nach Anspruch 1, zusätzlich enthaltend ein FSP, umfassend die folgende Aminosäuresequenz:
ASPKCIMKEKKSLVRLSSCVPVALMSAMTTSSSQKNITPAILTCC (TGFBR2 (-1)).

3. Impfstoff nach Anspruch 1 oder 2, wobei das FSP zusätzlich eine Tag-Sequenz enthält.

4. Impfstoff, enthaltend (i) die Nukleinsäuresequenzen, die für das FSP von einem der Ansprüche 1 bis 3 kodieren, oder (ii) Vektor/en, enthaltend die Nukleinsäuresequenzen.

5. Impfstoff nach Anspruch 4, wobei der Vektor ein Plasmid oder viraler Vektor ist.

6. Impfstoff nach Anspruch 5, wobei der virale Vektor ein auf einem Pockenvirus, Adenovirus, Retrovirus, Herpesvirus, Alphavirus beruhender Vektor oder adenoassoziiertes Virus (AAV) ist.

7. Impfstoff nach Anspruch 6, wobei das Pockenvirus ein Vaccinia-Virus, NYVAC, Vogelpockenvirus, Kanarienpockenvirus, ALVAC, ALVAC(2), Geflügelpockenvirus oder TROVAC ist.

8. Impfstoff nach einem der Ansprüche 1 bis 7, zusätzlich umfassend ein Adjuvans und/oder immunstimulatorisches Zytokin oder Chemokin.

9. Impfstoff nach einem der Ansprüche 1 bis 8, wobei die Wirkverbindung in einem Öl-in-Wasser- oder einem Wasser-in-Öl-Emulsionsträger dargestellt ist.

10. Impfstoff nach einem der Ansprüche 1 bis 9, zusätzlich umfassend ein oder mehrere Antigene.

11. Impfstoff nach einem der Ansprüche 1 bis 10 zur Verwendung in einem Verfahren zur Verhinderung oder Behandlung eines Tumors.

12. Verwendung der MSI-Tumor-spezifischen Frameshift-Peptide (FSP) nach einem der Ansprüche 1 bis 3, der Nukleinsäure nach Anspruch 4 oder des Vektors nach einem der Ansprüche 4 bis 7 zur Herstellung eines Impfstoffs zur Verhinderung oder Behandlung eines Tumors.

13. Impfstoff nach einem der Ansprüche 1-11 zur Verwendung nach Anspruch 11 oder 12 durch vorsorgliche Impfung von Hochrisikogruppen.

14. Impfstoff nach einem der Ansprüche 1-11 zur Verwendung nach einem der Ansprüche 11 bis 13, wobei der Tumor ein Kolorektalkrebs, Endometriumskrebs, Magenkrebs oder Dünndarmkrebs ist.

15. Impfstoff nach einem der Ansprüche 1-11 zur Verwendung nach Anspruch 14, wobei der Kolorektalkrebs ein erblicher Kolorektalkrebs ohne Polyposis (HNPCC) ist.

## Revendications

1. Vaccin contenant une combinaison de peptides à déphasage (FSP) spécifiques à MSI comprenant les séquences d'acides aminés suivantes:
NTQIKALNRGLKKKTILKKAGIGMCVKVSSIFFINKQKP (TAF1B (-1));
EIFLPKGRSNSKKKGRRNRIPAVLRTEGEPLHTPSVGMRETTGLGC (HT001(-1));
et
HSTIKVIKAKKKHREVKRTNSSQLV (AIM2 (-1)).

2. Vaccin selon la revendication 1, contenant en outre un FSP comprenant la séquence d'acides aminés suivante:
ASPKCIMKEKKSLVRLSSCVPVALMSAMTTSSSQKNITPAILTCC (TGFBR2 (-1)).

3. Vaccin selon la revendication 1 ou 2, dans lequel le FSP contient en outre une séquence marqueur.

4. Vaccin contenant: (i) les séquences d'acides nucléiques codant le FSP selon l'une quelconque des revendications 1 à 3 ou (ii) un ou des vecteurs contenant lesdites séquences d'acides nucléiques.

5. Vaccin selon la revendication 4, dans lequel ledit vecteur est un plasmide ou un vecteur viral.

6. Vaccin selon la revendication 5, dans lequel ledit vecteur viral est un poxvirus, un adénovirus, un rétrovirus, un herpèsvirus, un vecteur à base d'alphavirus ou un virus adéno-associé (AAV).

7. Vaccin selon la revendication 6, dans lequel ledit poxvirus est un virus de la vaccine, NYVAC, un avipox virus, le canarypox virus, ALVAC, ALVAC(2), le fowlpox virus ou TROVAC.

8. Vaccin selon l'une quelconque des revendications 1 à 7, comprenant en outre un adjuvant et/ou une cytokine ou chimiokine immunostimulante.

9. Vaccin selon l'une quelconque des revendications 1 à 8, dans lequel le composé actif est présenté dans un véhicule sous forme d'une émulsion huile-dans-eau ou eau-dans-huile.

10. Vaccin selon l'une quelconque des revendications 1 à 9, comprenant en outre un ou plusieurs autres antigènes.

11. Vaccin selon l'une quelconque des revendications 1 à 10 destiné à être utilisé dans un procédé pour la prévention ou le traitement d'une tumeur.

12. Utilisation des peptides à déphasage (FSP) spécifiques pour la tumeur MSI tels que définis dans l'une quelconque des revendications 1 à 3, de l'acide nucléique tel que défini dans la revendication 4 ou du vecteur tel que défini dans l'une quelconque des revendications 4 à 7 pour la préparation d'un vaccin pour la prévention ou le traitement d'une tumeur.

13. Vaccin selon l'une quelconque des revendications 1 à 11 destiné à être utilisé selon la revendication 11 ou 12 par vaccination préventive de groupes à haut risque.

14. Vaccin selon l'une quelconque des revendications 1 à 11 destiné à être utilisé selon l'une quelconque des revendications 11 à 13, où la tumeur est un cancer colorectal, un cancer de l'endomètre, un cancer de l'estomac ou un cancer du côlon.

15. Vaccin selon l'une quelconque des revendications 1 à 11 destiné à être utilisé selon la revendication 14, où le cancer colorectal est un cancer colorectal non-polypose héréditaire (HNPCC).
